# EUROPEAN PATENT SPECIFICATION

(11) **EP 1 245 149 B1**
(45) Date of publication and mention of the grant of the patent: **15.11.2006**
(21) Application number: 01107969.6
(22) Date of filing: 29.03.2001
(51) Int. Cl.: A01H 4/00, A01H 5/08, A01H 1/00, C12N 15/89, C12N 15/82

(54) **Plastid transformation of lycopersicon plants**
Plastidentransformation bei Lycopersicon
Transformation de plastides chez Lycopersicon

(43) Date of publication of application: 02.10.2002
(73) Proprietor: FRAUNHOFER-GESELLSCHAFT ZUR FÖRDERUNG DER ANGEWANDTEN FORSCHUNG E.V., 80686 München (DE)
(72) Inventor: Bock, Ralph, 48147 Münster (DE); Carrer, Helaine, Piracicaba, SP, 13416-790 (BR)
(74) Representative: Stürken, Joachim

(56) References cited:
- WO-A-00/20611
- WO-A-00/28014
- ZORA SVAB AND PAL MALIGA: "High-frequency plastid transformation in tobacco by selection for a chimeric aadA gene" PROC. NATL. ACAD. SCI. USA, vol. 90, 1993, pages 913-917, XP002190803
- Z. SVAB, P. HAJDUKIEWICZ, P. MALIGA: "Stabel transformation of plastids in higher plants" PROC. NATL. ACAF. SCI. USA, vol. 87, 1990, pages 8526-8530, XP002190804
- P.B. HEIFETZ: "Genetic engeneering of the chloroplast" SOCI¹T¹ FRANÇAISE DE BIOCHIMIE ET BIOLOGIE MOL¹CULAIRE, vol. 82, 2000, pages 655-666, XP002190805

## Description

The present invention relates to the field of genetically transforming plant plastids, and more specifically to genetically transforming the plastid genomes of plant material having the potential to regenerate into mature fertile plants belonging to the genus *Lycopersicon* (tomato).

### Background of the invention

Throughout this application various publications are referenced by citation within parantheses the complete cites for which may be found at the end of the specification immediately preceding the claims.

The plastids of higher plants are an attractive target for genetic engineering. Plant plastids (chlorophyll-containing chloroplasts, starch-storing amyloplasts, oil-containing elaioplasts, yellow, orange or red carotenoid-containing chromoplasts, and etioplasts being partially developed chloroplasts that form in dark-grown seedlings) are the major biosynthetic centers that, in addition to photosynthesis, are responsible for production of industrially important compounds such as amino acids, complex carbohydrates, fatty acids, pigments, and the like. Plastids are derived from a common precursor known as a proplastid and thus the plastids present in a given plant species all have the same genetic content. The plastid genome of higher plants is a circular double-stranded molecule of 120 to 160 kb harboring approximately 130 genes. Identical copies of this genome are present in all cells and all plastid types mentioned above. A most remarkable feature of the plastid genome is its extremely high ploidy level: A single tobacco leaf cell may contain as many as 100 chloroplasts with each harboring approximately 100 identical copies of the plastid genome resulting in an extraordinarily high ploidy degree of up to 10,000 plastid genomes per cell which potentially can result in very high levels of foreign gene expression.

The recent development of technologies to engineer the chloroplast genome of the green alga *Chlamydomonas reinhardtii* (1) and the higher plant *Nicotiana tabacum* (2) has opened up the possibility to target transgenes to the plastid genome by chloroplast transformation. These technologies offer a great potential for the biotechnology of the future (3, 4, 5) and a number of most attractive advantages over conventional transgenic plants (generated by transformation of the nuclear genome) such as, e.g.:
- High levels of transgene expression and foreign protein accumulation of up to more than 40% of the total soluble cellular protein (10-100 times higher than upon nuclear transgene expression), presumably due to the polyploidy of the plastid genetic system and/or the high stability of foreign proteins;
- Possibility of expressing multiple transgenes as operons ("transgene stacking") due to efficient translation of polycistronic mRNAs in plastids;
- Absence of position effects in plastids due to lack of a compact chromatin structure and efficient transgene integration via homologous recombination;
- Absence of epigenetic effects (gene silencing);
- Transgene containment due to uniparentally maternal inheritance of chloroplasts in most higher plants (i.e. absence of pollen transmission of transgenes).

In higher plants, chloroplast transformation is routinely available only in tobacco, *Nicotiana tabacum* (U.S. Patent Number 5,451,513). The main obstacle to extending the technology to other species and, most importantly, to major crops is probably posed by limitations in the currently available tissue culture systems and regeneration protocols for transplastomic plants. Although some progress was made recently with Arabidopsis and potato chloroplast transformation (9, 10), the production of fertile transplastomic plants in any other species but tobacco has not yet been reported. In fact, the three chloroplast transformants generated to date for the model plant *Arabidopsis thaliana* all were sterile and hence could not be propagated generatively (9). For review, see also L. Bogorad (17).

WO 0020611 A discloses transformation methods that provide for reproducible and efficient expression of a gene of interest from the genome of plant plastids by using DNA molecules comprising flanking DNA sequences that allow homologous recombination within the recipient.

WO 0028014 A discloses methods for transforming solanaceous plants to express DNA of interest from the plant cell plastid and states that this method can be utilized in the transformation of solanaceous plants, such as potato and petunia. Although the transformation of tobacco and potato plants is disclosed, no guidance is given as to the selection and regeneration of tomato plants for which a specific protocol had to be established.

It follows from the above that transgenic chloroplasts offer unique advantages in plant biotechnology, including high-level foreign protein expression, absence of epigenetic effects and gene containment due to the lack of transgene transmission through pollen. However, broad application of plastid genome engineering in biotechnology has been largely hampered by (i) the lack of chloroplast transformation systems for major crop plants and (ii) the usually low plastid gene expression levels in non-green tissues, such as fruits, tubers and other storage organs. (cf. eg. WO 0028014).

The present invention is based on the successful development of a novel plastid transformation/selection/regeneration system for the generation of fertile transplastomic plants in a food crop, first established for tomato, *Lycopersicon esculentum.* According to the principles of the invention, it could be shown that chromoplasts in the tomato fruit express a desired transgene to ~50% of the expression levels in leaf chloroplasts. Given the generally very high foreign protein accumulation rates that can be achieved in transgenic chloroplasts (more than 40% of the total soluble protein), this system paves the way to efficient production of edible vaccines, pharmaceuticals and antibodies in plants.

### Summary of the invention

The present invention provides methods for obtaining stably transformed homoplasmic transplastomic plant cell material belonging to the genus *Lycopersicon* and having the potential to regenerate into mature fertile plants. The method generally comprises (a) transforming plastids of said plant cell material with a DNA molecule carrying an expression cassette encoding at least one protein of interest, and a target sequence enabling homologous recombination; (b) selecting plant cell material until substantially all plastids have been transformed with said DNA molecule, thereby obtaining said stably transformed homoplasmic transplastomic plant cell material; and, optionally (c) regenerating said transplastomic plant cell material into mature homoplasmic fertile plants. Furthermore, the present invention provides stably transformed homoplasmic transplastomic *Lycopersicon* plant cells, seeds, tissues and organs having the potential to regenerate into mature fertile plants, as well as mature homoplasmic fertile plants, obtainable by carrying out the methods according to the invention, and use thereof for the production of proteinaceous substances.

### Brief description of the drawings

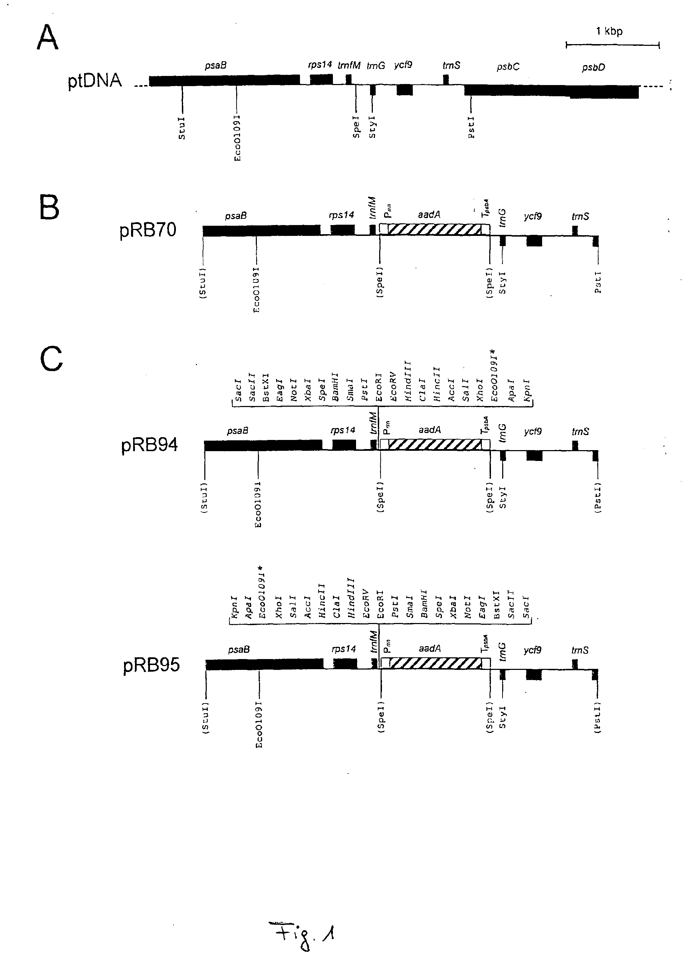

Figure 1 shows the construction of chloroplast transformation vectors containing polylinkers for convenient cloning of passenger genes. **(A)** Physical and restriction map of the chloroplast genome region used for construction of plasmid vectors for plastid transformation. **(B)** Map of the plastid transformation vector pRB70 containing a chimeric *aadA* gene driven by the rRNA operon promoter. **(C)** Map of vectors pRB94 and pRB95. The two plasmids carry the polylinker taken from pBluescript upstream of the *aadA* gene in different orientations. Several restriction sites for polylinker enzymes within the vector sequences were eliminated by mutageneses. As a result, almost all restriction sites present in the polylinker are unique (enzymes shown in italics) and thus can be used for insertion of passenger genes. Note that the EcoO109I site in the polylinker although not unique can be utilized for cloning since the second site within the *psaB* gene is Dcm methylated in *E. coli* and hence not recognized in plasmids prepared from standard (*dcm+*) laboratory strains. Restriction sites eliminated by mutagenesis or ligation of heterologous ends are shown in parentheses.

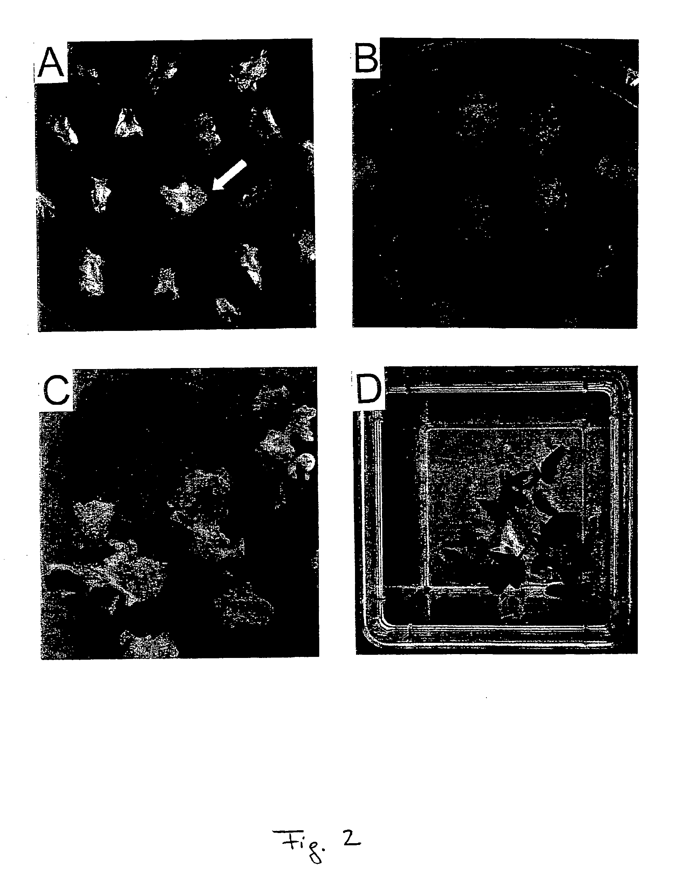

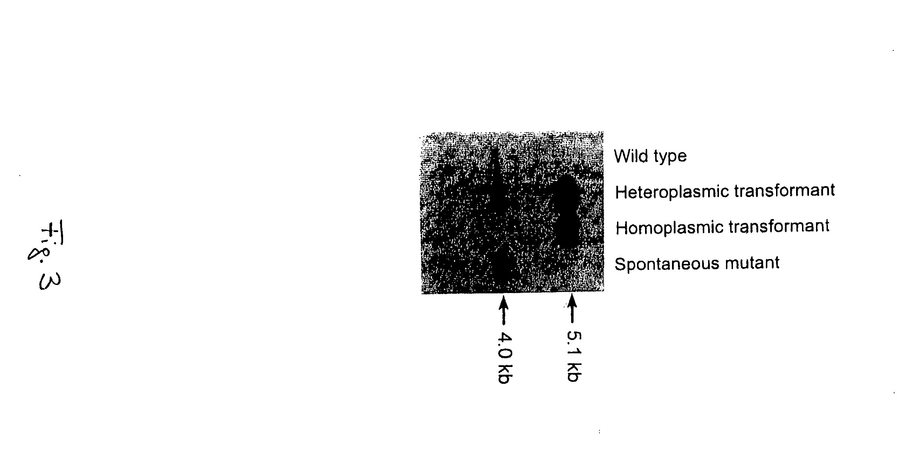

Figure 2 shows the generation of tomato plants with transgenic plastids. **(A)** Primary selection of spectinomycin-resistant tomato calli (selection phase A). A plate with bombarded leaf pieces is shown after 3 months incubation on spectinomycin-containing tissue culture medium. Note that the leaf tissue is bleached out due to effective inhibition of chloroplast translation. Spectinomycin-resistant calli appear as small yellow or pale green mounts of dividing cells (arrow). **(B)** Propagation of spectinomycin-resistant tomato lines. Tissue samples from primary plastid transformants are subjected to additional selection cycles on antibiotic-containing culture medium (selection phase B). On this medium, the tissue grows as undifferentiated callus from which samples are taken in regular intervals for homoplasmy tests (Fig. 3). **(C)** Plant regeneration from homoplasmic transplastomic callus tissue. Shoot regeneration is observed approximately 4 weeks after transfer of homoplasmic callus material to shoot induction medium. **(D)** Rooting of transplastomic tomato shoots. Shoots induced from homoplasmic calli are transferred to boxes with hormone-free rooting medium. Following successful rooting, plants are transferred to the soil and grown to maturity in the greenhouse.

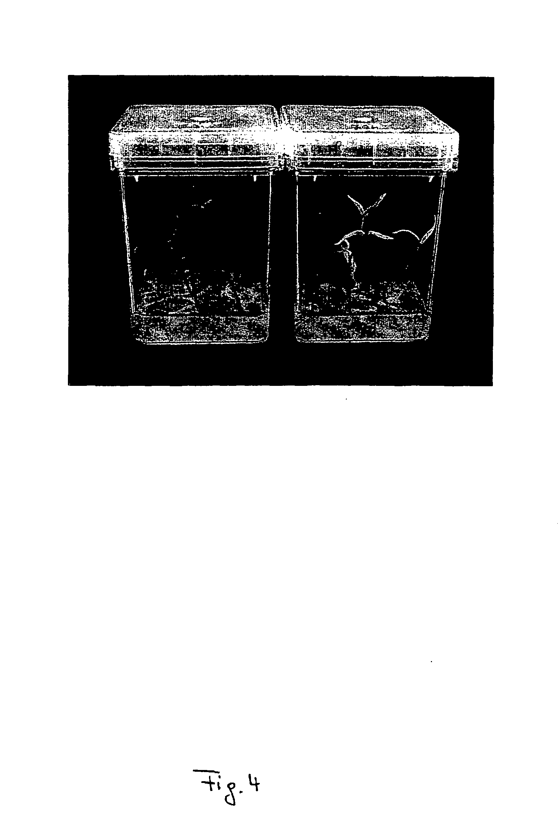

Figure 3 displays an RFLP analysis to identify homoplasmic transplastomic tomato lines. A wild-type sample, a spontaneous spectinomycin-resistant line, a heteroplasmic transplastomic line and a homoplasmic line are shown. DNAs were digested with EcoO109I and PstI and probed with a radiolabeld StyI/PstI restriction fragment (Fig. 1). To increase the sensitivity of the assay, approximately 5 times more DNA from the transplastomic lines was loaded. Whereas the heteroplasmic line clearly contains a mix of wild-type and transformed chloroplast genomes, even upon strong overexposure of the blot (not shown), no signal for the wild-type plastid genome could be detected in the homoplasmic line suggesting that, in three successive selection cycles, all wild-type plastid DNA molecules were successfully eliminated. Homoplasmy was additionally confirmed by uniparentally maternal inheritance of the plastid transgene (Fig. 4).
Figure 4 proves maternal inheritance of the spectinomycin resistance trait in the F1 progeny of transplastomic tomato plants. Flowers from transplastomic plants were pollinated with pollen from wild-type plants and the seeds were germinated on MS medium containing spectinomycin. Whereas the wild-type control is clearly spectinomycin sensitive and all seedlings bleach out (right), F1 seedlings from the cross of a transplastomic tomato plant with a wild-type plant exhibit uniform resistance to the antibiotic (left).

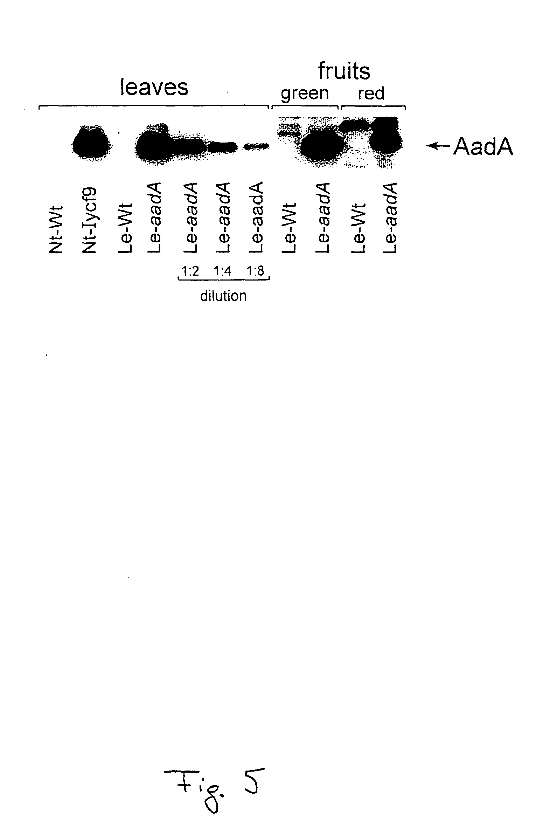

Figure 5 displays foreign protein accumulation in leaves, green and ripe red fruits of transplastomic tomato plants. Samples representing 15 µg of extracted total cellular proteins were electrophoresed in PAA gels, blotted to PVDF membranes and incubated with an AadA-specific polyclonal antibody. As controls, wild-type tomato samples were included for all tissues. For comparison, a transplastomic tobacco line harboring the identical chimeric *aadA* gene (Nt-Iycf9; (15)) as well as a dilution series of leaf proteins from a transplastomic tomato plant are shown. Note that minor cross-reacting bands in fruit protein extracts are common to wild-type and transplastomic plants and hence do not represent AadA protein.

### Detailed description of the invention

In accordance with the subject invention, methods are provided for obtaining stably transformed homoplasmic transplastomic plant cell material belonging to the genus *Lycopersicon* containing plastids into which foreign DNA has been inserted, and having the potential to regenerate into mature fertile plants. The term "foreign DNA" as used herein is meant to encompass all DNA sequences which are either homologous or heterologous with respect to the the wild-type plastid genome of the respective host desired to be transformed.

The methods generally encompass transforming a plant cell with plastid expression vectors. The plastid expression constructs generally contain nucleic acid sequences comprising, as operably linked components in the 5' to 3' direction of transcription, a promotor functional in a plant plastid, at least one DNA sequence of interest, and a 3' untranslated region capable of stabilizing transcripts in a plant plastid.

According to the invention, the methods generally comprise the steps of:
(a) transforming plastids of plant cell material belonging to the genus *Lycopersicon* with a DNA molecule enabling selection of transformed plant cell material and carrying an expression cassette encoding at least one protein of interest, and a target sequence enabling homologous recombination;
(b) selecting, propagating, and purifying the transformed plant cell material obtained in (a) to homoplasmy, wherein the selection comprises a primary (A) and a secondary (B) selection phase, wherein phase A comprises illuminating the plant cell material of step (a) under selection conditions using reduced average light intensities ranging between 2 and 55 micromole quanta per square meter and second (2-55 µE) until transplastomic callus tissue has been formed which can be transferred and regenerated, and wherein phase (B) comprises further propagation of the transformed plant cell material obtained in selection phase (A) and isolation of homoplasmic transplastomic tissue that lack any residual copies of the wild-type plastid genome, and, optionally
(c) regenerating said transplastomic plant cell material into mature homoplasmic fertile plants.

It is preferred, that the average light intensity applied ranges between 2 and 55 µE, more preferred between 20 and 30 µE, with a light intensity of approximately 25 µE being most preferred. The term "average" with respect to reduced light intensities is meant to encompass modifications of the methods according to the invention, wherein different µE values within the aforementioned range may be applied during a given illumination period.

With respect to the primary selection phase A, it is preferred to perform the same for at least 60 days, preferably for at least 80 days.

According to the principles of the present invention selection phases A and B are defined as follows. Phase A comprises selection of primary transplastomic plant material until callus tissue has been formed which can be transferred and regenerated, as exemplified by Fig. 2A, whereas phase B comprises propagation/multiplication of the transplastomic plant material as well as purification to homoplasmy, as exemplified by Fig. 2B.

As to the plant cell material subjected to transformation, essentially all plant parts and tissues that have been proposed in the literature can be selected, although it is preferred that the plant cell material subjected to transformation is derived from leaf tissue. Following transformation, it is particularly preferred that the transformed leaf material to be subjected to selection phase A has an average surface area between 6 and 16 mm², with a surface area of approximately 9 mm² being most preferred.

As will be appreciated by a person skilled in the art, the present invention reliably enables plastid transformation of plant cell material belonging to the genus *Lycopersicon,* which preferably is selected from the group consisting of *Lycopersicon esculentum, Lycopersicon pimpinellifolium, Lycopersicon cheesmanii, Lycopersicon hirsutum, Lycopersicon pennellii, Lycopersicon peruvianum, Lycopersicon chilense* and *Lycopersicon chmielewskii.*

According to another preferred aspect of the present invention, the expression cassette of the DNA molecule as used in step (a) is controlled by use of a promotor for the plastid rRNA-operon. These promotors are highly conserved and can easily be selected by an artisan.

Furthermore, stably transformed homoplasmic transplastomic *Lycopersicon* plant cell material including cells, seeds, tissues and organs having the potential to regenerate into mature fertile plants, obtainable by carrying out the above method according to the invention, as well as mature homoplasmic fertile plants regenerated from said stably transformed transplastomic plant cell material are provided, wherein said plant material is preferably selected from the group consisting of *Lycopersicon esculentum, Lycopersicon pimpinellifolium, Lycopersicon cheesmanii, Lycopersicon hirsutum, Lycopersicon pennellii, Lycopersicon peruvianum, Lycopersicon chilense* and *Lycopersicon chmielewskii.*

Additionally, the invention relates to the use of said stably transformed homoplasmic transplastomic *Lycopersicon* plant cells, seeds, tissues, organs and mature fertile plants for the production of proteinaceous substances, comprising cultivating the plant cell material or the mature fertile plants in order to accumulate said proteinaceous substances.

It is to understood, that various modifications to the methods exemplified according to the invention can be envisaged by the artisan without leaving the general principle set forth throughout the description. In particular, alternative constructs comprising other homologous or heterologous targeting sequences ensuring homologous recombination, different members of a broad range of suitable DNA sequences of interest and alternative selection markers as well as other transformation techniques can easily be applied to the present invention and, thus, are encompassed by the present invention. For example, reference is made to alternative means, such as disclosed in WO 97 32977, WO 0028014, and WO 0039313.

Expression constructs for use in the methods of the present invention find use in directing the expression of foreign DNA sequences encoding genes involved in a wide variety of plant genetic engineering applications. Such sequences may encode for proteins involved in agronomic traits (input traits) such as herbicide tolerance and disease resistance, or quality traits (output traits) such as fatty acid composition modification and carotenoid production. Furthermore, DNA sequences encoding for proteins for the production of human biologics in a plant cell plastid also find use in the expression constructs or cassettes of the present invention.

The skilled artisan will recognize that other DNA sequences may be utilized in the constructs for use in the methods according to the invention. In addition, the expression cassettes also find use in directing the production of human biological proteins (pharmaceutical proteins) from the plant plastid.

As to the use of selectable markers or reporters it is to be understood that the presence of DNA sequences encoding suitable markers or reporters in the expression cassettes may be ommitted in cases where the foreign DNA sequences code for proteins or polypeptides which themselves can serve as selectable markers.

Reflecting the unexpected results obtained by carrying out the invention, it is to be noted, that the advantages over the state of the art using biolistic transformation techniques are primarily achieved by (i) the use of extreme low-light conditions during the primary selection phase A, (ii) the drastic extension of the primary selection phase A to at least 60 days compared with 3-5 weeks in tobacco, (iii) the significantly smaller size of the leaf pieces exposed to the tissue culture medium during primary selection as compared to tobacco plastid transformation (Fig. 2A), and (iv) the optimization of the selection and plant regeneration scheme (see below). In particular, successful selection of transplastomic tomato cells was found to be critically dependent on the use of much lower light intensities (e.g. 25 µE) than for selection of transplastomic tobacco plants (70-100 µE). As will be appreciated by an artisan, the above items (iii) and/or (iv) would have to be modified in case of applying alternative transformation protocols.

Transgene expression levels in the consumable parts of the plant (which frequently are non-green) are central to the wide use of plastid transformation technologies in biotechnology. Most endogenous plastid genome-encoded genes are involved in photosynthesis and hence are drastically down-regulated in non-photosynthetic tissues. Ripe tomato fruits contain chromoplasts, a carotenoid-accumulating differentiation type of plastids. Chloroplasts are present in green tomatoes and are then converted into chromoplasts during the fruit ripening process. Chromoplasts in tomato fruits were shown to carry out active protein biosynthesis and to contain large amounts of plastid ribosomal RNAs. For this reason, an rRNA operon-derived chimeric promoter (8) was used to drive transgene expression in tomato plastid transformation. Using an AadA protein-specific antibody, foreign protein accumulation in leaves, green fruits and ripe red tomatoes were compared. Unexpectedly, high expression levels (approximately half of the expression levels in green leaves) are achieved even in red tomatoes (Fig. 5). Also, foreign protein accumulation does not change significantly during the ripening process, presumably due to the ribosomal rRNA operon promoter conferring more or less constitutive expression of the transgene.

The unexpected finding, that high levels of foreign protein accumulation in transplastomic tomato plants are not confined to photosynthetically active tissue but also occur in ripe fruits, opens up new applications for transplastomic technologies, such as the efficient production of nutraceuticals and biopharmaceuticals in plants.

Accordingly, the present invention additionally provides effective methods for the production of desired proteinaceous substances by using transplastomic plant material obtained according to the transformation/selection/regeneration protocols disclosed herein.

As already mentioned, transplastomic technologies offer a tremendous potential for the production of safer and more nutritious foods. With the successful development of a plastid transformation protocol for tomato as provided by the present invention, there is now a first system established which (i) allows plastid genome engineering in a crop for human consumption, (ii) results in production of fertile transplastomic plants and (iii) yields high-level foreign protein expression in consumable plant organs. Although earlier work with Arabidopsis (9) and potato (10) has resulted in successful chloroplast transformation, all transplastomic Arabidopsis lines were male and female sterile and could only be propagated vegetatively in tissue culture (9). Likewise, the generation of fertile transplastomic potato plants and uniparental transgene transmission to the next generation has not yet been reported.

In transplastomic potato plants, foreign protein accumulation was found to be 100-fold lower in non-photosynthetic microtubers than in green leaves (10). At present, it can only be speculated why transgene expression is so much higher in tomato fruits than in potato microtubers. One reasonable explanation could be that the tomato fruit consisted of green, photosynthetically active tissue before initiation of the ripening process and that active plastid gene expression is maintained upon conversion of chloroplasts to chromoplasts during fruit ripening. In fact, tomato chromoplasts are known to carry out active protein biosynthesis and contain large amounts of plastid ribosomal RNAs whereas the mRNA levels of most photosynthesis-related genes were found to be drastically down-regulated. In this respect, the choice of chimeric rRNA operon-derived promoters to drive transgene expression in the tomato fruit may be ideal to achieve high expression levels. However, there is likely room for further improvement: Combination of the rRNA operon promoter with the bacteriophage gene 10 leader and a sequence encoding the first 14 amino acids of the green fluorescent protein (GFP) yielded a dramatic increase in transgene expression in transplastomic tobacco (18). Moreover, recent experiments with Bt toxin expression in tobacco have demonstrated that foreign protein accumulation in leaves of transplastomic plants can reach values of more than 45% of the total soluble protein of the cell (7). Extrapolating from these data, one might expect that at least 20% foreign protein accumulation can be reached in fruits of transplastomic plants which would be 10 times more than what maximally can be achieved with nucleocytoplasmic transgene expression.

Clearly, plastid transformation in tomato is currently much more laborious and time consuming than plastid transformation in the two only well-established standard objects, tobacco and Chlamydomonas. The first successful plastid transformation experiments with tomato took almost two years from the biolistic bombardment of the leaves up to the harvest of the first ripe transplastomic tomatoes. Certainly, the procedure can be further optimized without departing from the principles of the present invention.

The following examples are illustrative but not limiting of the present invention.

### Examples

### Construction of plastid transformation vectors

In chloroplast transformation experiments with tobacco, numerous regions of the chloroplast genome were tested for being suitable target sites for the uptake of transgenes. In the course of the eperiments which led to the present invention, a region in the chloroplast genome (Fig. 1A) was identified, that, when used in transformation vectors as targeting sequence for homologous recombination, resulted in particularly high chloroplast transformation frequencies. Accordingly, this region was chosen and a series of plastid transformation vectors was derived from it. In these constructs, as will be described in more detail below, the selectable marker gene *aadA* was inserted in between two tRNA genes (Fig. 1B, C). For the convenient insertion of passenger genes and their tight linkage to the selectable spectinomycin resistance gene *aadA*, the pBluescript® (Stratagene) polylinker was inserted in different orientations immediately upstream of the *aadA* marker (Fig. 1C).

The *rps14*/*trnfM* region (Fig. 1A) was cloned from the tobacco plastid genome as a 3.4 kb PstI/StuI fragment into pBluescript® (Stratagene) (PstI/Ec1136II). A chimeric *aadA* gene driven by the rRNA operon promoter (8) was inserted into the unique SpeI site in between the genes for tRNAGly and tRNAfMet after blunting the SpeI site by a fill-in reaction with Klenow enzyme. For subsequent manipulations, a clone was selected in which the *aadA* cassette has the same orientation as the upstream *trnfM* gene (plasmid pRB70; Fig. 1B). The remaining polylinker was eliminated by digestion of pRB70 with Apal and PstI followed by blunting with mung bean nuclease and religation (clone pRB83). Remaining restriction sites for polylinker enzymes within the aadA cassette (KpnI, SpeI, XbaI) were removed by mutagenesis, fill-in reactions with Klenow enzyme or mung bean nuclease treatment. The complete polylinker from pBSII SK was PCR amplified with M13 primer and reverse primer and the PCR product was cloned into a HincII site in between *trnfM* and *aadA*. Clones were selected for both polylinker orientations and control sequenced (plasmids pRB94 and pRB95; Fig. 1C).

The plastid targeting region in the transformation vectors is highly conserved in the chloroplast genomes of dicotyledonous plants and hence is expected to be suitable not only for plastid transformation in the genus tomato but also for other higher plants.

### Plant material

Sterile tomato plants (*Lycopersicon esculentum* var. "Santa Clara") were raised in Magenta boxes (double-boxes with a connector element, Magenta Corp., Illinois) from surface-sterilized seeds germinated on MS medium (12). Homoplasmic transplastomic plants and wild-type control plants were transferred to the soil and grown to maturity in a phytochamber (16 hours light, 8 hours dark, 24 °C). Leaf and fruit material for analysis of nucleic acids and proteins was frozen in liquid nitrogen and stored at -70°C until use. Control tobacco plants were grown under identical conditions.

### Plastid transformation and regeneration of homoplasmic transplastomic tomato plants

Plastid transformation of tomato was achieved by biolistic bombardment of young sterile tomato leaves with gold particles of 0.6 µm diameter coated with plasmid pRB70 DNA using the DuPont PDS1000He biolistic gun (2, 13) and 1100 psi rupture disks (BioRad). Bombarded leaf samples were cut into small pieces having an average surface area of approximately 9 mm², transferred to RMOP medium containing spectinomycin (300-500 mg/l; (2, 8)) and incubated under dim light (25 µE; 16 hours light, 8 hours dark) for a time period of 3 to 4 months, depending on the time of appearance of transferable antibioticresistant callus tissue. Primary spectinomycin-resistant lines were identified as yellow or pale green growing calli (Fig. 2A) and appeared to be very light-sensitive at this stage. Callus pieces were transferred to the same but fresh selection medium for further propagation and isolation of homoplasmic transplastomic tissue that lack any residual copies of the wild-type plastid genome (phase B)(2). Unlike tobacco, tomato tissue did not show shoot development on this medium and kept growing as green calli (Fig. 2B). At this stage, successful chloroplast transformation was verified using analyses set forth below in more detail. An initial fast test by PCR identified chloroplast transformants and allowed elimination of spontaneous spectinomycin-resistant lines (Table 1). After one to two additional cycles of callus propagation on the identical medium, chloroplast transformation and homoplasmy were ultimately confirmed by RFLP analyses (Fig. 3). As the *aadA* gene confers broad-range resistance to a variety of antibiotics of the aminoglycoside type (8), chloroplast transformants were additionally tested for double resistance to both spectinomycin and streptomycin. Whereas spontaneous spectinomycin-resistant lines are sensitive to streptomycin and bleach out on tissue culture medium containing these two drugs, continued callus growth of transplastomic tissue provided further evidence of successful chloroplast transformation and efficient expression of the plastid *aadA* marker.

For plant regeneration, homoplasmic callus tissue was transferred onto the surface of agar-solidified shoot induction (MS) medium containing 0,2 mg/l indole-3-acetic acid (IAA) as auxin and 3 mg/l 6-benzylaminopurine (BAP) as cytokinin. Alternatively, shoot induction was obtained with the same medium but 2 mg/l zeatin instead of BAP. For subsequent rooting, regenerated shoots were transferred into boxes containing phytohormone-free MS medium (Fig. 2D). The resulting transplastomic plants were then planted in the soil and grown in the greenhouse to maturity.

### DNA extraction, PCR and RFLP analyses

Total cellular DNA was extracted using a CTAB-based method (14). For RFLP analysis, DNA samples were digested with either PstI and XhoI or PstI and EcoO109I (Fig.1), electrophorezed in 1 % agarose gels and blotted onto nylon membranes (Hybond® N, Amersham/Pharmacia). RFLPs and homoplasmy of transplastomic plants were detected by subsequent hybridization to a radiolabeled StyI/PstI restriction fragment (Fig. 1) in RapidHyb® buffer following the instructions of the supplier (Amersham/Pharmacia). PCR reactions were performed according to standard protocols (45s at 94°C, 1.5 min at 55 °C, 1.5 min at 72 °C; 30 cycles) using primer pairs specific for the chimeric *aadA* gene.

### Crosses and tests of maternal transgene inheritance

To confirm uniparentally maternal transgene transmission to the next generation, emasculated flowers from transplastomic plants were pollinated with pollen from wild-type plants. To test the transmission of the resistance trait, surface-sterilized F1 seeds were germinated on MS medium containing 100 mg/l spectinomycin. Transmission of the *aadA* resistance gene was monitored by the green seedling phenotype and continued growth and development in the presence of the antibiotic in contrast to bleaching and ceased growth of sensitive progeny.

As expected for a plastid-encoded trait, the F1 progeny resulting from these crosses was uniformly spectinomycin-resistant (Fig. 4) confirming both stable transformation of the tomato plastid genome and homoplasmy of the transplastomic lines.

A single bombarded tomato leaf sample typically resulted in eight to nine selection plates with leaf pieces on the surface of spectinomycin-containing callus induction medium (Table 1). In three independent transformation experiments, 6 tomato chloroplast transformants were selected altogether, equaling an transformation efficiency of one transplastomic line selected from approximately 80-100 selection plates. Although this efficiency is significantly lower than the plastid transformation frequency in the well-established tobacco system (where routinely one chloroplast transformant per 5-10 selection plates is obtained), tomato plastid transformation is efficient enough to provide a workable and attractive system for both basic research and plant biotechnology.

**Table 1**

| **Experiment No** | **Bombarded Leaf Samples** | **Number of Selection Plates** | **Spectinomycin- resistant Calli** | **PCR Positive** | **RFLP Positive** |
|---|---|---|---|---|---|
| 1 | 20 | 180 | 6 | 3 | 3 |
| 2 | 20 | 180 | 3 | 1 | 1 |
| 3 | 20 | 160 | 4 | 2 | 2 |

Table 1 displays the statistics of three independent chloroplast transformation experiments in tomato. Calli displaying resistance to spectinomycin but being negative in PCR and RFLP tests are likely to be spontaneous resistance mutants which arise through acquisition of point mutations in the 16S rRNA gene (16).

### Protein extraction and immunoblot analyses

Total soluble protein from the various tissues was extracted from samples homogenized in a buffer containing 300 mM sucrose, 50 mM Tris/HCl, 10 mM EDTA, 2 mM EGTA, 10 mM DTT and 1 mM Pefabloc® (Roche Diagnostics)(using a mortar and adding washed and calcined fine granular quartz). The homogenates were filtered through two layers of Miracloth® (Calbiochem) followed by centrifugation at 12,000 rpm for 8 min. For each tissue, samples representing 15 µg of extracted proteins were separated by polyacrylamide gel electrophoresis and blotted to PVDF membranes using a standard tank blotter (BioRad). Membranes were subsequently incubated with an AadA-specific polyclonal antibody (raised in rabbits and kindly provided by Dr. Jean-David Rochaix, University of Geneva, Switzerland) followed by detection with the Western Blot Chemiluminescence Reagent Plus system (NEN).

### Plastid transgene expression in tomato leaves and fruits

The promoter used to drive plastid transgene expression in tomato is the strong, more or less constitutively expressed ribosomal RNA operon promoter *Prrn* (8). In tobacco, this promoter was shown to confer foreign protein accumulation of up to 5% of the total soluble leaf protein (6) and in an extreme case even more than 45% (7). In order to evaluate whether the protein accumulation levels in transplastomic tomato plants would be similar to those in transplastomic tobacco, comparative analysis of foreign protein accumulation levels in transplastomic tomato plants and in tobacco plants carrying the identical chloroplast transgene were performed (Fig. 5). No significant difference was found indicating that plastid transgenes in tomato are expressed to similarly high levels as in tobacco.

Taking advantage of the high transgene expression levels in tomato fruit chromoplasts, the system provided herein can now be used to introduce new agronomically and biotechnologically relevant traits into tomato plants by plastid transformation. Current experiments in which a first set of passenger genes along with the *aadA* selectable marker gene is introduced into the tomato plastid genome have already been performed. The data obtained demonstrate successful plastid transformation and stable integration of these passenger genes into the plastid DNA.

As will be appreciated by a person skilled in the art, the availability of a technology for transgene expression from the tomato plastid genome according to the principles of the present invention will open up new possibilities for metabolic engineering, resistance management and the use of plants as factories for biopharmaceuticals. Plants have considerable potential for the production of edible vaccines, antibodies ("plantibodies") and therapeutic substances (for a recent review see e. g. 11). For such applications, plastid transformation technologies according to the invention or derived therefrom do not only offer solutions to the technical and ecological problems associated with conventional transgenic technologies (such as transgene silencing, outcrossing etc.) but also provide the advantage that much higher transgene expression levels can be achieved.

### References cited

1. Boynton, J. E., Gillham, N. W., Harris, E. H., Hosler, J. P., Johnson, A. M., Jones, A. R., Randolph-Anderson, B. L., Robertson, D., Klein, T. M., Shark, K. B. & Sanford, J. C. Science 240, 1534-1538 (1988).
2. Svab, Z., Hajdukiewicz, P. & Maliga, P. Proc. Natl. Acad. Sci. USA 87, 8526-8530 (1990).
3. Daniell, H. Trends Plant Sci. 4, 467-469 (1999).
4. Hager, M. & Bock, R. Appl. Microbiol. Biotechnol. 54 302-310 (2000).
5. Heifetz, P. B. Biochimie 82, 655-666 (2000).
6. McBride, K. E., Svab, Z., Schaaf, D. J., Hogan, P. S., Stalker, D. M. & Maliga, P. Bio/Technology 13, 362-365 (1995).
7. De Cosa, B., Moar, W., Lee, S.-B., Miller, M. & Daniell, H. Nature Biotechnol. 19, 71-74 (2001).
8. Svab, Z. & Maliga, P. Proc. Natl. Acad. Sci. USA 90, 913-917 (1993).
9. Sikdar, S. R., Serino, G., Chaudhuri, S. & Maliga, P. Plant Cell Rep. 18 20-24 (1998).
10. Sidorov, V. A., Kasten, D., Pang, S.-Z., Hajdukiewicz, P. T. J., Staub, J. M. & Nehra, N. S. Plant J. 19, 209-216 (1999).
11. Giddings, G., Allison, G., Brooks, D. & Carter, A. Nature Biotechnol. 18, 1151-1155 (2000).
12. Murashige, T. & Skoog, F. Physiol. Plant. 15, 493-497 (1962).
13. Kanevski, I. & Maliga, P. Proc. Natl. Acad. Sci. USA 91, 1969-1973 (1994).
14. Doyle, J. J. & Doyle, J. L. Focus 12, 13-15 (1990).
15. Ruf, S., Biehler, K. & Bock, R. J. Cell Biol. 149, 369-377 (2000).
16. Svab, Z. & Maliga, P. Mol. Gen. Genet. 228, 316-319 (1991).
17. Borogard, L. TIBTECH 18, 257-263 (2000).
18. Ye, G.-N., Hajdukiewicz, P. T. J., Broyles, D., Rodriguez, D., Xu, C. W., Nehra, N. & Staub, J. M. Plant J., in press (2001).

## Claims

1. A method for obtaining stably transformed homoplasmic transplastomic plant cell material belonging to the genus *Lycopersicon* and having the potential to regenerate into mature fertile plants, comprising the steps of:
(a) transforming plastids of said plant cell material with a DNA molecule enabling selection of transformed plant cell material and carrying an expression cassette encoding at least one protein of interest, and a target sequence enabling homologous recombination;
(b) selecting, propagating, and purifying the transformed plant cell material obtained in (a) to homoplasmy, wherein the selection comprises a primary (A) and a secondary (B) selection phase, wherein phase A comprises illuminating the plant cell material of step (a) under selection conditions using reduced average light intensities ranging between 2 and 55 micromole quanta per square meter and second (2-55 µE) until transplastomic callus tissue has been formed which can be transferred and regenerated, and wherein phase (B) comprises further propagation of the transformed plant cell material obtained in selection phase (A) and isolation of homoplasmic transplastomic tissue that lack any residual copies of the wild-type plastid genome, and, optionally
(c) regenerating said transplastomic plant cell material into mature homoplasmic fertile plants.

2. The method according to claim 1, wherein phase A is conducted for at least 60 days.

3. The method according to claim 1 or 2, wherein the plant cell material subjected to transformation is derived from leaf tissue.

4. The method according to claim 3, wherein the transformed leaf material subjected to selection phase A has an average surface area between 6 and 16 mm².

5. The method according to any of the preceding claims, wherein the plant cell material belonging to the genus of *Lycopersicon* is selected from the group consisting of *Lycopersicon esculentum, Lycopersicon pimpinellifolium, Lycopersicon cheesmanii, Lycopersicon hirsutum*, *Lycopersicon pennellii, Lycopersicon peruvianum*, *Lycopersicon chilense* and *Lycopersicon chmielewskii.*

6. The method according to any of the preceding claims, wherein the expression cassette of the DNA molecule as used in step (a) is controlled by a promoter for the plastid rRNA-operon.

7. Stably transformed homoplasmic transplastomic *Lycopersicon* plant cell material including cells, seeds, tissues and organs having the potential to regenerate into mature fertile plants, obtainable by carrying out the method according to any of claims 1 to 6, as well as mature homoplasmic fertile plants regenerated from said stably transformed transplastomic plant cell material.

8. Stably transformed homoplasmic transplastomic *Lycopersicon* plant cells, seeds, tissues, organs, and mature fertile plants regenerated therefrom, according to claim 7, in which the expression cassette is controlled by a promoter for the plastid rRNA-operon.

9. Stably transformed homoplasmic transplastomic *Lycopersicon* plant cells, seeds, tissues, organs, and mature fertile plants according to claim 7 or 8, selected from the group consisting of *Lycopersicon esculentum*, *Lycopersicon pimpinellifolium*, *Lycopersicon cheesmanii*, *Lycopersicon hirsutum*, *Lycopersicon pennellii, Lycopersicon peruvianum*, *Lycopersicon chilense* and *Lycopersicon chmielewskii.*

10. Use of the stably transformed homoplasmic transplastomic *Lycopersicon* plant cells, cells, seeds, tissues and organs having the potential to regenerate into mature fertile plants, as well as mature fertile plants regenerated therefrom, as defined in any of claims 7 to 9, for the production of proteinaceous substances, comprising cultivating the plant cell material or the mature fertile plants in order to accumulate said proteinaceous substances.

## Patentansprüche

1. Verfahren zum Erhalt stabil transformierten homoplasmischen transplastomischen Pflanzenzellmaterials, welches zur Gattung *Lycopersicon* gehört und das Potential besitzt, zu reifen fertilen Pflanzen zu regenerieren, umfassend die Schritte:
(a) Transformation von Plastiden des Pflanzenzellmaterials mit einem DNA-Molekül, welches eine Selektion transformierten Pflanzenzellmaterials ermöglicht und eine für mindestens ein interessierendes Protein kodierende Expressionskassette sowie eine Zielsequenz trägt, die homologe Rekombination ermöglicht;
(b) Selektion, Vermehrung, und Aufreinigung des in (a) erhaltenen transformierten Pflanzenzellmaterials zur Homoplasmie, wobei die Selektion eine primäre (A) und eine sekundäre (B) Selektionsphase umfasst, wobei Phase A die Beleuchtung des Pflanzenzellmaterials von Schritt (a) unter Selektionsbedingungen unter Verwendung von reduzierten durchschnittlichen Lichtintensitäten in einem Bereich zwischen 2 und 55 Mikromol Quanten pro Quadratmeter und Sekunde (2-55 µE) umfasst, bis transplastomisches Kallusgewebe gebildet worden ist, welches transferiert und regeneriert werden kann, und wobei Phase (B) die weitere Vermehrung des in Selektionsphase (A) erhaltenen transformierten Pflanzenzellmaterials und die Isolierung homoplasmischen transplastomischen Gewebes umfasst, welches frei ist von jeglichen restlichen Kopien des Wildtyp-Plastidengenoms, und, gegebenenfalls
(c) Regeneration des transplastomischen Pflanzenzellmaterials zu reifen homoplasmischen fertilen Pflanzen.

2. Verfahren nach Anspruch 1, wobei Phase A für mindestens 60 Tage durchgeführt wird.

3. Verfahren nach Anspruch 1 oder 2, wobei das der Transformation zugeführte Pflanzenzellmaterial von Blattgewebe abgeleitet ist.

4. Verfahren nach Anspruch 3, wobei das der Selektionsphase A zugeführte transformierte Blattmaterial eine durchschnittliche Oberfläche zwischen 6 und 16 mm² aufweist.

5. Verfahren nach irgendeinem der vorhergehenden Ansprüche, wobei das zur Gattung *Lycopersicon* gehörende Pflanzenzellmaterial ausgewählt wird aus der Gruppe bestehend aus *Lycopersicon esculentum, Lycopersicon pimpinellifolium, Lycopersicon cheesmanii, Lycopersicon hirsutum, Lycopersicon pennellii, Lycopersicon peruvianum, Lycopersicon chilense* und *Lycopersicon chmielewskii.*

6. Verfahren nach einem der vorhergehenden Ansprüche, wobei die Expressionskassette des in Schritt (a) verwendeten DNA-Moleküls von einem Promotor für das plastidäre rRNA-Operon kontrolliert wird.

7. Stabil transformiertes homoplasmisches transplastomisches *Lycopersicon* Pflanzenzellmaterial einschließlich Zellen, Samen, Gewebe und Organe mit dem Potential zur Regeneration zu reifen fertilen Pflanzen, erhältlich durch Ausführung des Verfahrens gemäß irgendeinem der Ansprüche 1 bis 6, sowie reife homoplasmische fertile Pflanzen, die aus dem stabil transformierten transplastomischen Pflanzenzellmaterial regeneriert wurden.

8. Stabil transformierte homoplasmische transplastomische *Lycopersicon* Pflanzenzellen, Samen, Gewebe, Organe, und davon regenerierte reife fertile Pflanzen, nach Anspruch 7, in denen die Expressionskassette von einem Promotor für das plastidäre rRNA-Operon kontrolliert wird.

9. Stabil transformierte homoplasmische transplastomische *Lycopersicon* Pflanzenzellen, Samen, Gewebe, Organe, und reife fertile Pflanzen nach Anspruch 7 oder 8, ausgewählt aus der Gruppe bestehend aus *Lycopersicon esculentum, Lycopersicon pimpinellifolium, Lycopersicon cheesmanii, Lycopersicon hirsutum, Lycopersicon pennellii, Lycopersicon peruvianum*, *Lycopersicon chilense* und *Lycopersicon chmielewskii.*

10. Verwendung der stabil transformierten homoplasmischen transplastomischen *Lycopersicon* Pflanzenzellen, Samen, Gewebe und Organe mit dem Potential zur Regeneration zu reifen fertilen Pflanzen, sowie davon regenerierte reife fertile Pflanzen, gemäß irgendeinem der Ansprüche 7 bis 9, zur Herstellung proteinöser Substanzen, umfassend die Kultivierung des Pflanzenzellmaterials oder der reifen fertilen Pflanzen zur Akkumulation der proteinösen Substanzen.

## Revendications

1. Une méthode pour l'obtention du matériel cellulaire de plante transformé de manière stable, homoplasmique, transplastomique, appartenant au genre *Lycopersicon* et possédant le potentiel de régénérer en plantes matures fertiles, comprenant les étapes de:
(a) transformation des plastides du dit matériel cellulaire de plante avec une molécule d'ADN permettant la sélection du matériel cellulaire de plante transformé et portant une cassette d'expression codant pour au moins une protéine d'intérêt et une séquence cible permettant une recombinaison homologue;
(b) sélection, propagation et purification du matériel cellulaire de plante transformé obtenu en (a) jusqu'à l'homoplasmie, dans lesquelles la sélection comprend une phase de sélection primaire (A) et secondaire (B), où la phase A comprend l'illumination du matériel cellulaire de plante de l'étape (a) sous des conditions de sélection utilisant des intensités de lumière moyenne réduites comprises entre 2 et 55 micromoles quanta par mètre carré et seconde (2-55 µE) jusqu'à ce que du tissu calleux transplastomique se soit formé, pouvant être transféré et régénéré, et où la phase (B) comprend la propagation ultérieure du matériel cellulaire de plante transformé obtenu dans la phase de sélection (A) et l'isolation du tissu homoplasmique transplastomique qui ne contient aucune copie résiduelle du génome plastidaire sauvage, et, en option,
(c) régénération du dit matériel cellulaire de plante transplastomique en des plantes matures homoplasmiques fertiles.

2. Procédé selon la revendication 1 dans lequel la phase A est poursuivie pendant au moins 60 jours.

3. Procédé selon la revendication 1 ou 2 dans lequel le matériel cellulaire de plante soumis à la transformation est dérivé de tissu foliaire.

4. Procédé selon la revendication 3 dans lequel le matériel foliaire transformé soumis à la phase A de sélection a une surface moyenne comprise entre 6 et 16 mm².

5. Procédé selon une des revendications précédentes dans lequel le matériel cellulaire de plante appartenant au genre *Lycopersicon* est sélectionné dans le groupe consistant en *Lycopersicon esculentum, Lycopersicon pimpinellifolium, Lycopersi con cheesmanii, Lycopersi con hirsu tum, Lycopersi con pennellii, Lycopersicon peruvanium, Lycopersicon chilense* et *Lycopersicon chmielewskii.*

6. Procédé selon une des revendications précédentes dans lequel la cassette d'expression de la molécule d'ADN utilisée dans l'étape (a) est contrôlée par un promoteur pour l'opéron des rRNA plastidaires.

7. Matériel cellulaire de plante appartenant au genre *Lycopersicon,* transformé de manière stable, homoplasmique, transplastomique, y compris les cellules, les graines, les tissus et les organes possédant le potentiel de régénérer en plantes matures fertiles, obtenu en appliquant la méthode selon une des revendications 1 à 6, ainsi que des plantes matures homoplasmiques fertiles régénérées du dit matériel cellulaire de plante transplastomique transformé de manière stable.

8. Cellules de plante, graines, tissus, organes, appartenant au genre *Lycopersicon,* transformés de manière stable, transplastomiques, homoplasmiques, et plantes matures fertiles régénérées de ceux-ci, selon la revendication 7, dans lesquels la cassette d'expression est contrôlée par un promoteur pour l'opéron de rRNA plastidaire.

9. Cellules de plante, graines, tissus, organes, appartenant au genre *Lycopersicon,* transformés de manière stable, transplastomiques, homoplasmiques, et plantes matures fertiles, selon la revendications 7 ou 8, sélectionnés dans le groupe consistant en *Lycopersicon esculentum, Lycopersicon pimpinelli folium, Lycopersicon cheesmanii*, *Lycopersicon hirsutum, Lycopersicon pennellii, Lycopersicon peruvanium, Lycopersicon chilense* et *Lycopersicon chmielewskii.*

10. Utilisation de cellules de plante, graines, tissus et organes appartenant au genre *Lycopersicon,* transformés de manière stable, transplastomiques, homoplasmiques, possédant le potentiel de régénérer dans des plantes matures fertiles, ainsi que des plantes matures fertiles régénérées de ceux-ci, comme défini dans une des revendications 7 à 9, pour la production de substances protéagineuses, y compris la culture de matériel cellulaire de plante ou de plantes matures fertiles pour pouvoir accumuler les dites substances protéagineuses.
